# EUROPEAN PATENT APPLICATION

(11) **EP 3 486 917 A1**
(43) Date of publication of application: **22.05.2019**
(21) Application number: 17425117.3
(22) Date of filing: 17.11.2017
(51) Int. Cl.: G16H 40/20

(54) **METHOD FOR MANAGING MANAGEMENT PROCESSES AND WORKGROUPS DEPLOYED IN MULTI-SITE AMBULATORY HEALTH FACILITIES**

(71) Applicant: CMO S.r.l., 80133 Napoli (IT)
(72) Inventor: Del Prete, Antonio, 73100 Lecce (LE) (IT); Capalbo, Vincenzo, 73100 Lecce (LE) (IT); Marulo, Luigi, 80133 Napoli (NA) (IT)
(74) Representative: Conversano, Gabriele

(57) **Abstract**

Computer software for managing working processes in multi-site health-care centers, comprising:
(i) a project management module,
(ii) a workflow managing module,
(iii) a multi-user communication model,
said project management module being configured to send information about creation and users authentication to said workflow managing module and to said communication module;
said workflow managing module being configured to contain working processes models and to allow users to modify the same,
and said multi-user communication module being configured to allow users to interact.

## Description

### Field of the invention

The present invention relates to a method for organizing and managing knowledge-based processes and supporting cooperative work in project management oriented organizations, and in particular for managing management processes and document filing in multi-site health-care centers. The current working reality usually exploits working models based on teams belonging to the companies and structured by skills on a functional basis, comprising groups of multi-skilled experts, distributed at various organizational structures, whose collaboration is focused on reaching a specific target, and therefore, is time-limited. Typically, these teams of experts collaborate on the development of a new product/service, help carry out a project or provide a certain service, and do not stay in the team for the whole duration of the collaboration but they go in and out from the team according to the project needs (service). These organizational models are considered extremely efficient at favouring innovation since they allow to develop products and services focusing on the customer needs. Flexibility, little use of hierarchy and informal communication mechanisms between managers and workers guarantee complex horizontal interactions which are the main source of innovation. While the most part of vertical companies is approaching project-oriented and process-oriented models, the fast increase in information and communications technologies have helped make emerge and impose what has been defined *network organization*, and which is turning out to be efficient both in manufacturing and services. The present invention provides a method which, by means of the combination of workflows management tools with a web-based management project tool and a multi-user communication system builds a Collaborative Working Environment (Collaborative Working Environment or CWE).

In particular, the features of CWE implementing the method according to the invention are such that management processes, document modification and filing are optimized and made easier, with particular reference to multi-site heath-care centers, whose peculiarity is the need to provide access to a plurality of kinds of documents and users located in different facilities. Moreover, users who can have access to documents are of different kinds (typically doctors or anyway "health-care" users or administrative staff) and so, they must have a differentiated access level to the various document kinds.

While being implemented for managing this complexity, CWE becomes a document research tool substantial for managing and using company knowledge, as well as for managing health-care and administrative documentation concerning all the users of the facility.

### State of the art

Online collaboration, made easier by means of tools and collaborative working environments, favours a common knowledge among working groups and brings them to reach common targets. The Collaborative Working Environment (CWE) definition means a working environment where participants can collaborate by means of technological tools which allow to communicate synchronously (by means of conferences or instant messaging, etc.) or asynchronously (such as for example calendar, scheduler, email, forum etc.) for working activities development.

Small and medium-sized businesses prefer using these tools hosted in server of the same tool producer; while large businesses need to install them on own servers. So, there arises the problem of data safety and information transfer since such a working method enables to share knowledge and its re-usage between knowledge workers belonging to various organizations.

The feature of the current CWE is that they are not really integrated and inter-operational, but they are defined only for structured environments. As a consequence, they have a limited impact on innovativity and innovation. Organizations are in constant research for both innovative applications and services for improving business processes. Therefore aim of the present invention is to provide a directly usable solution, which allows to overcome the limits linked to the approaches known at the state of the art. In particular, the present invention provides useful elements for defining a Collaborative Working Environment able to support a dynamic management of processes oriented to favour collaboration among human actors and to guarantee autonomy and full transparency of operational activities.

The CWE according to the present invention manages a plurality of company processes in health-care centers, which are divided in:
- processes for managing requests to administrative staff: reservation, admission and report delivery processes;
- diagnostic processes requiring the action of users with high specialization profile to obtain the result or intended output, in this case the report specifying the diagnosis of the doctor;
- management processes and document filing.

### DESCRIPTION OF THE INVENTION

The invention provides a software implementing a method for managing processes of management and interaction among working processes comprising users with different profiles and distributed in a plurality of multi-site, polyclinic health-care centers, and in particular for document managing and filing, as well as electronic means on which said application is installed.

The application comprises an application layer and a layer containing data. The application layer contains:
(i) a project management module, from which information about creation and users authentication are sent to a workflow managing module and to a communication module;
(ii) a workflow managing module, wherein processes models are provided,
(iii) a multi-user communication model, configured to allow users to interact with the project management module and with the workflow managing module.

These modules are preferably web-base implemented, such that users located in different facilities can use them, provided that the facilities are connected to the network.

The workflow managing module is configured to allow users to define a plurality of processes models, which are saved in a dedicated database for the workflow managing module; the project management module is configured actually to carry out, case by case, the processes defined in the workflow managing module. The application comprises also a dedicated database for the project management module.

The multi-user communication module allows users to access, according to predetermined access levels, to the workflow managing modules and project management modules.

Therefore, the application comprises a dedicated database for the multi-user communication module. By using the just described structure, the software application is able to manage:
1. the configuration of the company processes;
2. the configuration of taxonomies:
   a. concerning the company resources skills;
   b. concerning operational cases of company interest;
3. the configuration of processes and profiles of users;
4. the planning of company cases as a function of the processes constituting them;
5. the creation of working groups for carrying out specific company activities (cases);
6. the beginning, monitoring and closing of company cases in the system;
7. the evaluation of team, by comparing the performance shown for assigned company cases;
8. the document managing and filing, user-centred, in presence inside the DB dedicated to the project management module of a section called "files" where all the diagnostic results and, more generally, the documents referred to each single customer are provided.

With specific reference to this last point, conveniently the database contains a section "files" where, for each customer, a plurality of documents, for example concerning the results of diagnostic tests carried out over time are contained.

The documents contained in the database are accessible by users also remotely authenticated by using the multi-user communication module, which allows each user to access, according to his own access privileges, to the documents of the database.

The system is able to manage the different access level (visualization only, creation of a new document, modification of existing document) to documents concerning a specific customer, by the users of the system, as a function of the profile of the same users.

Conveniently, the system is also able to provide the company documentation to carry out qualitative and quantitative analyses on rendered services.

In particular for each kind of documents it is possible to identify, for example, professionals who participated to writing the same and the workers who accomplished the administrative practices, and it will be possible to determine times spent between users requests and the end of the process, in addition to check the presence of possible double workings (caused for example by errors of the first draft of health-care and administrative documents).

In this way it is possible to define a plurality of indicators, also of statistic kind, able to monitor the efficiency and the quality of implemented working processes.

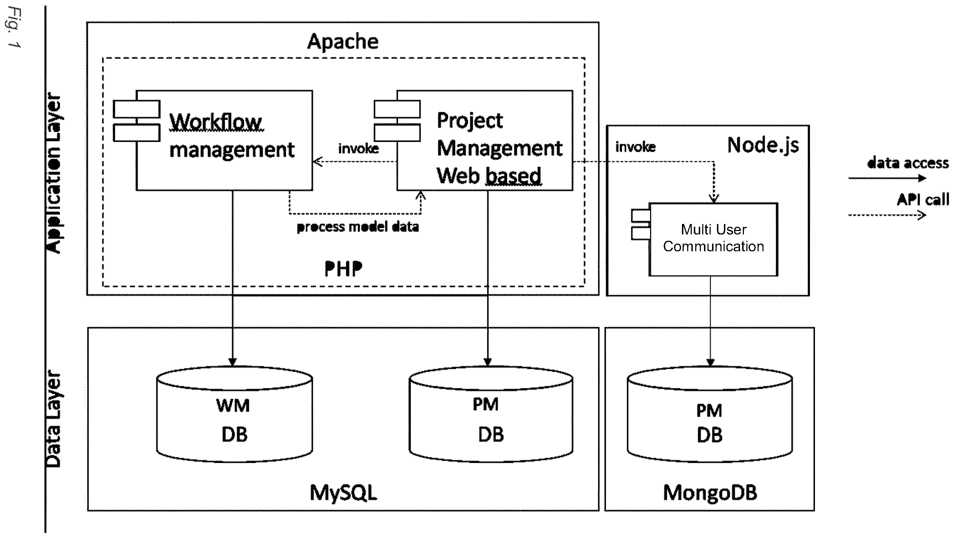

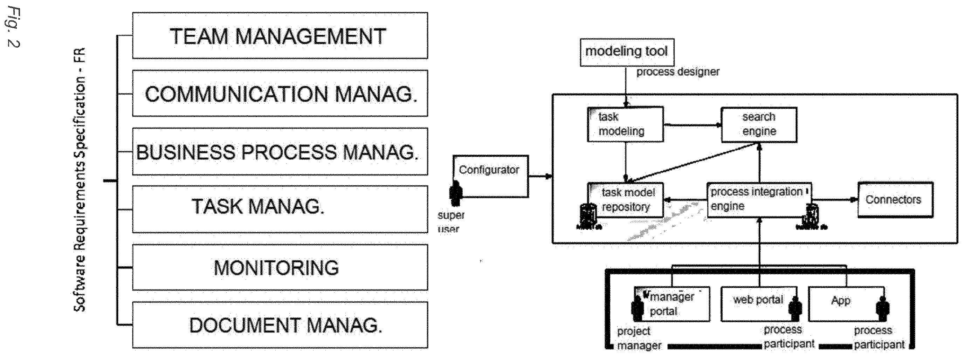

## Claims

1. Computer software for managing working processes in multi-site health-care centers, comprising:
(i) a project management module,
(ii) a workflow managing module,
(iii) a multi-user communication model,
said project management module being configured to send information about creation and users authentication to said workflow managing module and to said communication module;
said workflow managing module being configured to contain working processes models and to allow users to modify the same,
and said multi-user communication module being configured to allow users to interact with said workflow managing module and project management module.

2. Computer software for managing working processes in health-care centers according to claim 1, further comprising a dedicated database for each one of said modules.

3. Computer software for managing working processes in health-care centers according to claim 1 or 2, **characterized in that** the database concerning the project management module contains a plurality of documents concerning services carried out for each customer of the facility, and **in that** said documents are accessible by users by using said multi-user communication module.

4. Computer software for managing working processes in health-care centers according to claim 3, **characterized in that** said multi-user communication module allows each user to access to all or a portion of said documents as a function of the access level of each user.

5. Computer software for managing working processes in health-care centers according to any one of the preceding claims, **characterized in that** said project management module is configured actually to carry out, case by case, the working processes defined in the workflow managing module.

6. Computer software for managing working processes in health-care centers according to any one of the preceding claims, **characterized in that** said modules are preferably web-base implemented, such that users located in different facilities can use them, provided that the facilities are connected to the network.

7. Computer software for managing working processes in health-care centers according to any one of the preceding claims, **characterized in that** said program is configured so that it carries out qualitative and quantitative analyses on services rendered by said health-care centers.

8. Computer software for managing working processes in health-care centers according to claim 7, **characterized in that** said qualitative and quantitative analyses are carried out to determine times spent between users requests and the end of the process, and to check the presence of possible double workings and to define a plurality of indicators able to monitor the efficiency and the quality of implemented working processes.
